# EUROPEAN PATENT APPLICATION

(11) **EP 3 269 333 A1**
(43) Date of publication of application: **17.01.2018**
(21) Application number: 17180657.3
(22) Date of filing: 11.07.2017
(51) Int. Cl.: A61F 2/30, A61F 2/32, A61F 2/34, B29C 67/00

(54) **DEVICE FOR PROSTHESES AND RELATED PROCESS OF REALIZATION**

(30) Priority: 11.07.2016 IT 201600072345
(71) Applicant: Dimension 4 S.r.l., 06012 Città di Castello (PG) (IT)
(72) Inventor: BUCCI, Andrea, 06012 Città di Castello (PG) (IT)
(74) Representative: Brunacci, Marco

(57) **Abstract**

The process for the realization of a device for prostheses comprising:
- at least a bone integration portion (2) having an osseointegration surface (3) and a fixing surface (4), and adapted to be implanted in a skeletal structure;
- at least a contact portion (6) associated with the fixing surface (4) and having a contact surface (6a) adapted to engage with a joint element for the reconstruction of the joint of a patient;
with the integration portion (2) that comprises anchoring means (7) adapted to retain said contact portion (6) for their irreversible fixing;

comprises:
- a first stage of realization of a bone integration portion (2) of a device (1) for prostheses having an osseointegration surface (3) and a fixing surface (4);
- a second stage of realization of a contact portion (6) of the device (1) associated with said fixing surface (4) in an integral manner;

where the second stage is subsequent to the first stage.

## Description

The present invention relates to a device for prostheses and related process for its realization.

In medicine the use is known of prostheses for the partial or total replacement of injured skeletal elements.

To replace joints, use is made of prosthetic implants adapted to replace injured bone portions to restore the patient's joint mobility.

These implants provide for the use of prosthetic devices to be implanted for the replacement of the bone parts which originally formed the articulated joint.

In general, such devices are associable the one with the other to recreate the articulated joint, placed in contact in a movable way the one with respect to the other so as to recreate the joint movement.

The modular devices have an implant portion intended to be implanted in the bone and a contact portion intended to come into contact with the portion of the other device which is part of the prosthetic implant used to make the new articulated joint.

The portion of the implant is usually and generally made of titanium, and is fitted in the bone with the aim of fixing it to it by means of osseointegration processes.

The contact portion is usually made of a ceramic material, or other material with low friction coefficient (e.g. polyethylene) so as to favor reciprocal sliding with the other contact portion without developing degradation processes due to friction.

For example, in the case of hip prostheses, prosthetic implants are usually used comprising a reception device adapted to replace the acetabulum, and an insertion device adapted to replace the femoral head.

The reception device is usually a metal cup having an internal ceramic insert, while the insertion device has a substantially spherical shape.

The insertion device is adapted to be inserted in the reception device and is mobile with respect to it so as to recreate the articulated joint of the hip, thus permitting the typical movements of this joint.

The prosthetic implants used to replace the other joints (shoulders, ankles, elbows, etc.) are identical, with at least two devices associable with one another so as to recreate the respective articulated joint and allow its relative movements.

A first type of device used in the prosthetic implants are the "modular" devices.

The modular devices have an implant portion separated from the contact portion, joined together by means of the screwing of specific sealing elements or other fixing operations.

One drawback of this first type of device is tied to the non-perfect seal of the two portions.

The sealing elements, in fact, suffer gradual failure with consequent mobilization of one portion with respect to the other.

This drawback permits possible infiltrations of debris or other material between the portions of the device causing problems of various kinds to the patient.

The presence of bone debris or other material, in fact, not only negatively affects the good operation of the bone implant, but can cause the occurrence of autoimmune syndromes.

A second type of device is described in patent document US9248020 and envisages the device being made with two or more portions making up a single block, thus making it possible in part to overcome the drawbacks described above.

In particular, the aforementioned patent document illustrates a prosthesis comprising a device with a contact portion having a ceramic joint surface and an insertion portion having an osseointegration surface.

The two portions are associated with one another to form a monobloc prosthesis.

The bone contact portion is chemically tied to the ceramic portion and consequently we have a single block meant to be inserted directly in the bone. The same patent document also illustrates the process whereby the above-described prosthesis can be made.

In particular, such process envisages the prefabrication of the ceramic portion, the activation of a surface of such portion with oxygen and the subsequent covering of such activated surface with melted metal.

On cooling, the metal takes hold on the activated surface and bonds with it.

One drawback of this second type of device is tied precisely to the realization process.

The covering with melted metal on surface activated with oxygen, in fact, does not ensure a perfect seal between the metal portion and the ceramic portion of the end product.

This involves the continuation of the risk of infiltration of bone material or the like between the portions with consequent problems tied to the occurrence of autoimmune syndromes or to the gradual malfunction of the prosthetic implant.

The described process, furthermore, can only be adapted with difficulty to the production of prostheses personalized according to the anatomic requirements of individual patients.

The main aim of the present invention is to provide a device for prostheses and related process for its realization which permits making a device which is able to minimize the risk of infiltration of debris or other material between the various portions.

One object of the present invention is to provide a device for monobloc prostheses which facilitates the osseointegration processes.

Another object is to provide a process for the realization of the above device which allows simple and quick adaptations based on the personal needs of the individual patient.

Another object of the present invention is a device for prostheses and related process for its realization which allows to overcome the aforementioned drawbacks of the prior art within the ambit of a simple, rational, easy, efficient to use and cost-effective solution.

The aforementioned objects are achieved by this device for prostheses having the characteristics of claim 11 and related process for its realization having the characteristics of claim 1.

Other characteristics and advantages of the present invention will become more evident from the description of a preferred, but not exclusive, embodiment of a device for prostheses and related process for its realization, illustrated by way of an indicative, but non-limiting example, in the attached drawings in which:

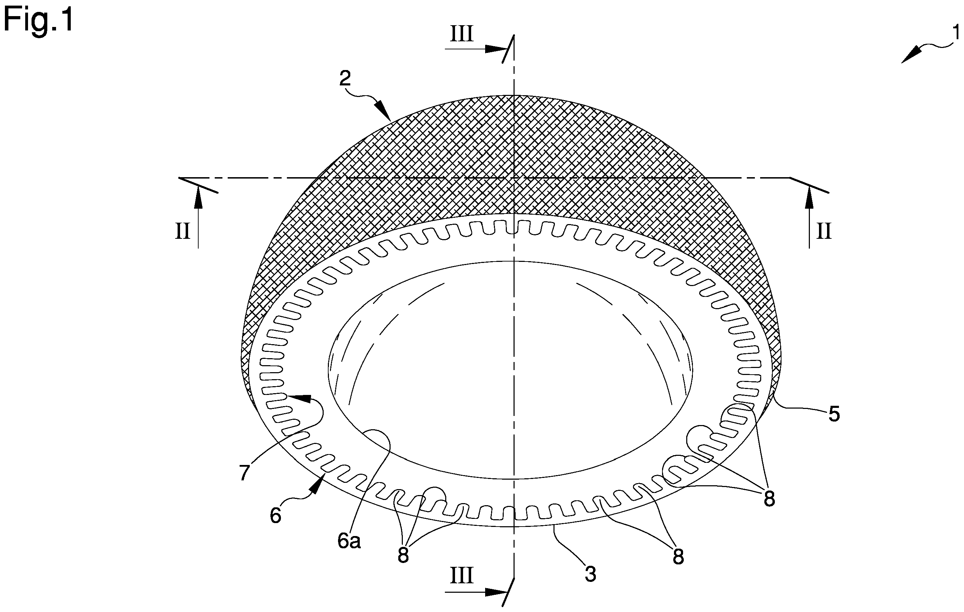

Figure 1 is an axonometric view of the device according to the invention;

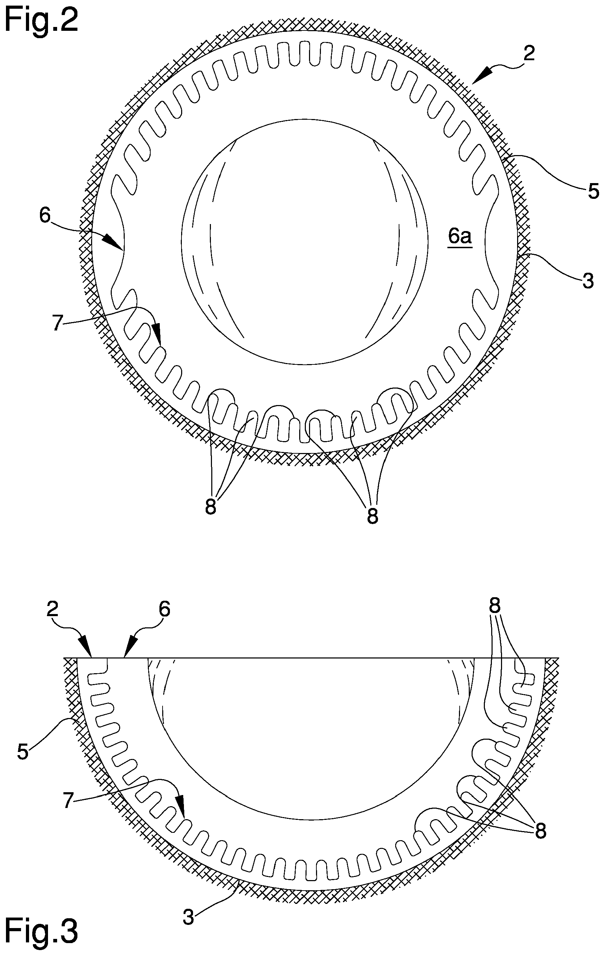

Figures 2 and 3 are sectional views of the device according to the invention;

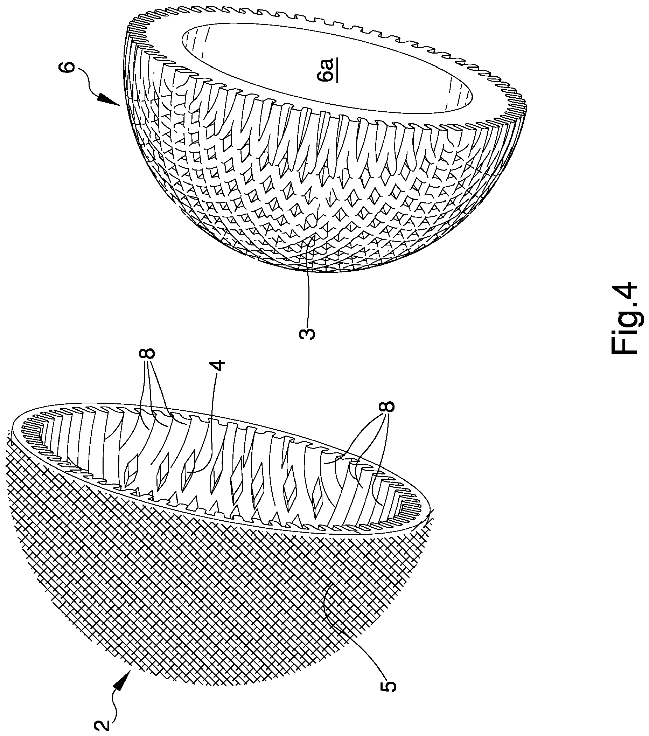

Figure 4 is an exploded axonometric view of the device according to the invention;
Figure 5 is a schematic illustration of some stages of the process for making the device according to the invention.

With particular reference to these figures, reference numeral 1 globally indicates a device for prostheses.

The device 1 shown in the illustrations is particularly adapted to be used in prosthetic implants used to replace the hip joint, but different embodiments cannot be ruled out, adapted to be used, for example, to replace other joints, such as the shoulder or ankle, or to replace other skeletal parts.

The device 1 comprises a bone integration portion 2 having an osseointegration surface 3 and a fixing surface 4, and adapted to be implanted in a skeletal structure.

The integration portion 2 is meant to be inserted directly in the bone of a patient to be treated and its function is to integrate with this to replace the original bone portion of the joint to be reconstructed.

Advantageously, the integration portion 2 comprises osseointegration means adapted to attract bone-growing cells.

In particular, the osseointegration surface 3 comprises protrusions 5 of bone integration arranged at a predefined distance to one another.

The protrusions 5, schematically shown in the figures, have a regular shape. The protrusions 5 have the same shape predefined on the basis of a digitalized model.

Advantageously, each protrusion 5 is provided with at least one microscopic cavity, but embodiments cannot be ruled out wherein the protrusions 5 have different shapes, e.g., branched.

Such characteristics make it possible to give the osseointegration surface 3 a regularized roughness, having an improved capacity to recall biological fluids containing bone re-growth cells compared to traditional rough surfaces. Preferably, the integration portion 2 is made of titanium, but the use of other materials, compatible with osseointegration, cannot be ruled out.

The device 1 also comprises at least a contact portion 6 associated with the fixing surface 4 and having a contact surface 6a adapted to engage with a joint element, for sake of simplicity not shown in the figures, for the reconstruction of the joint of a patient.

In this embodiment, which refers to a device to be used in prosthetic hip implants, the joint element is a second prosthetic device implanted in the thigh-bone of the patient to be treated, in particular to replace the femoral head.

In the case of prosthetic devices for other joints, the second device will be of different type, suitable for the joint involved in the surgery operation.

It cannot be ruled out that the joint element is a skeletal portion of the patient which is adapted to come into contact with the device 1.

The contact portion 6 is made of a ceramic material and is fixed to the fixing surface 4.

In particular, the contact surface 6a is entirely engaged with the fixing surface 4. According to the invention, the integration portion 2 comprises anchoring means 7 adapted to retain the contact portion 6 for their irreversible fixing, i.e. so as to obtain a coupling between the two portions 2, 6 which can be released only by means of the breakage of at least one of the two.

In particular, the anchoring means 7 comprise retaining undercuts 8 made on the fixing surface 4 and adapted to retain the contact portion 6.

Solutions cannot be ruled out which provide for anchoring means 7 having other elements adapted to make an anchorage in conjunction with the undercuts 8, or alternatively to them.

In the present embodiment, the undercuts 8 are longitudinal elements which extend overhanging with respect to the fixing surface 4 and trace on this groups of regular curves which intersect one another.

The empty spaces between the undercuts 8 and the fixing surface 4 are entirely occupied by the ceramic material.

This way, the fixing of the contact portion 6 to the integration portion 2 is improved.

The realization process of a device 1 for prostheses is as follows.

According to the invention, the process comprises a first stage, for sake of simplicity not shown in the figures, for the realization of a bone integration portion 2 of a device 1 for prostheses having an osseointegration surface 3 and a fixing surface 4.

Still according to the invention, the process comprises a second stage for the realization of a contact portion 6 of the device 1 associated with the fixing surface 4 in an integral manner, with the second stage which is subsequent to the first stage.

In particular, the first stage is constituted by a three-dimensional print of the integration portion 2.

The integration portion 2 is made with metallic material, preferably titanium.

The integration portion 2 is made using a 3D printer adapted to make the metal piece on the basis of information received from a three-dimensional digital model previously processed by means of specific software and comprising the desired geometric characteristics.

The integration portion 2, therefore, is first of all designed as a three-dimensional digital model, giving to the latter the desired geometric characteristics, and then printed on the basis of the same model.

This way, an integration portion 2 can be obtained, the geometry of which is engineered, i.e., has predefined, including complex characteristics, only obtainable by means of automatic design software, e.g., of the CAD type.

One example of these obtainable characteristics concerns the shape of the integration portion 2, which can be adapted to the characteristics of the patient, giving the device 1 the particular feature of a "custom made" device. Furthermore complex-geometry elements can be introduced and made.

In this regard, the three-dimensional print comprises a first stage of realization of anchoring means 7 adapted to retain the contact portion 6.

In particular, the first stage comprises shaping the fixing surface 4 with a plurality of undercuts 8.

The undercuts 8 are made to form longitudinal elements extending overhanging with respect to the fixing surface 4.

The undercuts 8 extend so as to form groups of regular curves on the fixing surface which intersect one another.

Different embodiments cannot be ruled out which provide for undercuts 8 shaped differently, e.g., to form segments which are spaced apart the one from the other.

After making the integration portion 2, the second stage can start.

The second stage comprises forming a provisional portion 12 of integration with melting material 9, adapted to be associated with the fixing surface 4.

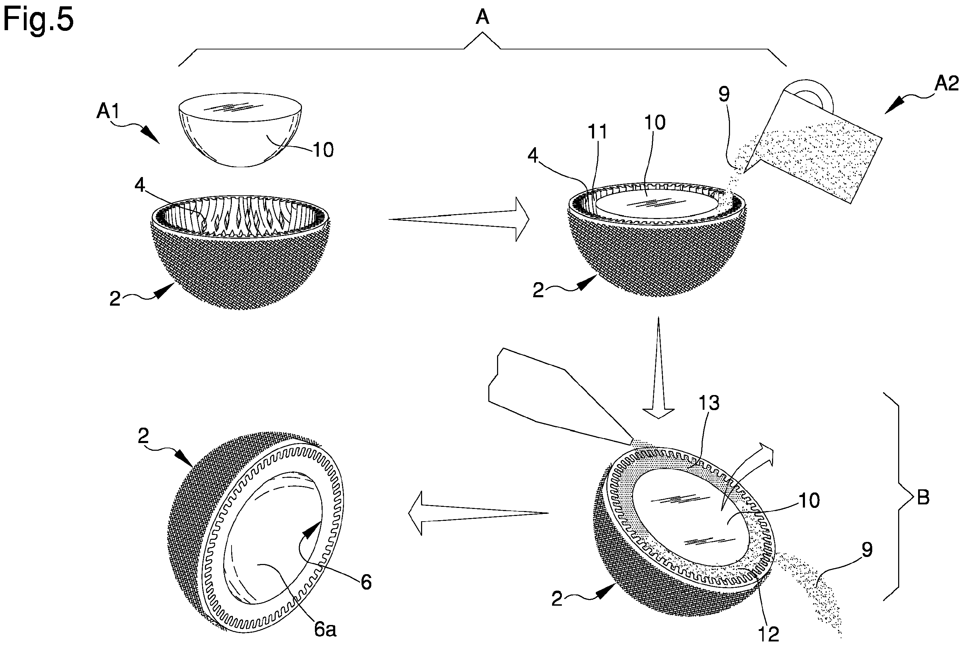

Such stage is shown in figure 5 and is indicated with reference letter A. Usefully, the melting material 9 is of the wax type, but the use cannot be ruled out of different materials as long as these are able to have a thermoplastic behavior.

In the present embodiment, the fact of forming a provisional portion comprises:
- inserting A1 a forming support 10 adapted to define a contact space 11 adjacent to the fixing surface 4;
- pouring A2 the melting material 9 in the liquid state in the contact space 11; and
- cooling the poured melting material 9 to obtain the provisional portion 12.

The forming support 10 shown in the illustrations is of the type of an embossed stamp shaped like a spherical cap, but different shapes cannot be ruled out according to the shape of the part of the joint affected by the prosthetic implant. The forming support 10 is smaller in size than the space enclosed by the integration portion 2 and the space that remains between the support and the fixing surface 4 is filled with the melted wax.

All this is cooled so as to convey to the melted wax an own conformation for the formation of the provisional portion 12.

The second stage then comprises the sub-stage of replacing the provisional portion 12 with contact material 13 adapted to form the contact portion 6.

This sub-stage is shown in figure 5 and is indicated with reference letter B. Preferably, the contact material 13 is of the type of ceramic material, having good sliding and electrical insulation properties as well as mechanical strength. Other types of material having similar properties cannot be ruled out.

In particular, the fact of replacing the provisional portion 12 comprises:
- heating the provisional portion 12 to obtain the melting of the melting material 9;
- inserting the contact material 13 to replace the melting material 9; and
- a heat treatment of the contact material 13 inserted to obtain the contact portion 6.

In this embodiment, the integration portion 2 with the provisional portion 12 undergoes heating so as to melt the wax 9 and allow it to be gradually moved away.

Meanwhile, the ceramic material 13 is inserted under pressure in the space left by the lost wax 9, and goes to replace the latter.

Once the replacement of the wax 9 by the ceramic material 13 has been made, the latter undergoes a heat treatment aimed at giving the ceramic material itself the properties of strength and solidity useful for definitively obtaining the contact portion 6.

The ceramic material 13, which in liquid state had penetrated the spaces between the undercuts 8 and the fixing surface 4, after solidifying creates an irreversible coupling, only separable by means of the breakage of at least one of the contact portion 6 and the integration portion 2.

It has in practice been ascertained how the described invention achieves the intended objects and in particular the fact is underlined that the provided device reduces the risk of infiltration of debris or other material between the various portions.

In fact, the particular fixing surface of the osseointegration portion, and in particular the undercuts obtained on it, allow for an inseparable coupling between the integration portion and the contact portion, without the possibility of micro movements between the two portions.

This way, the risk is reduced of their creating debris infiltrations between the two portions, and the functionality of the prosthesis is improved.

The process used to make the device, furthermore, permits both conveying the geometric characteristics needed to make the coupling between the contact portion and integration portion, and to make simple and quick adaptations based on the personal requirements of the individual patient.

Three-dimensional printing can in fact make contact portions the geometry of which is obtained directly from a digital model which is personalized according to the conformation of the skeletal portion of the patient.

This way "custom-made" devices can be quickly and simply reproduced, i.e., which are personalized according to the patient's bone morphology.

Finally, the provided process allows making a device for monobloc prostheses which facilitates osseointegration processes.

Three-dimensional printing does in fact allow making an osseointegration surface with engineered geometry, designed beforehand on a digital model and having geometric characteristics which favor the attraction of the biological fluids responsible for osseointegration.

In this respect, the provided device has an osseointegration surface with protrusions, the geometry of which is specially designed and printed to increase the attraction capacity of the aforementioned biological fluids.

## Claims

1. A process for the realization of a device for prostheses, **characterized by** the fact that it comprises:
- a first stage of realization of a bone integration portion (2) of a device (1) for prostheses having an osseointegration surface (3) and a fixing surface (4);
- a second stage of realization of a contact portion (6) of said device (1) associated with said fixing surface (4) in an integral manner;
said second stage being subsequent to said first stage.

2. The process according to claim 1, **characterized by** the fact that said first stage is constituted by a three-dimensional print of said integration portion (2).

3. The process according to one or more of the preceding claims, **characterized by** the fact that said three-dimensional print comprises a first stage of realization of anchoring means (7) adapted to retain said contact portion (6).

4. The process according to one or more of the preceding claims, **characterized by** the fact that said first stage of realization of anchoring means (7) comprises shaping said fixing surface (4) with a plurality of undercuts (8).

5. The process according to one or more of the preceding claims, **characterized by** the fact that said integration portion (2) is made with metallic material.

6. The process according to one or more of the preceding claims, **characterized by** the fact that said second stage comprises:
- forming (A) a provisional portion (12) of integration with melting material (9) and adapted to be associated with said fixing surface (4);
- replacing (B) said provisional portion (12) with contact material (13) adapted to form said contact portion (6).

7. The process according to one or more of the preceding claims, **characterized by** the fact that said melting material (9) is of the type of wax.

8. The process according to one or more of the preceding claims, **characterized by** the fact that said contact material (13) is of the type of ceramic material.

9. The process according to one or more of the preceding claims, **characterized by** the fact that said forming a provisional portion (12) comprises:
- inserting (A1) a forming support (10) adapted to define a contact space (11) adjacent to said fixing surface (4);
- pouring (A2) said melting material (9) in the liquid state in said contact space (11);
- cooling said poured melting material (9) to obtain said provisional portion (12).

10. The process according to one or more of the preceding claims, **characterized by** the fact that said replacing (B) comprises:
- heating said provisional portion (12) to obtain the melting of said melting material (9);
- inserting the contact material (13) to replace said melting material (9);
- a heat treatment of at least said contact material (13) inserted to obtain said contact portion (6).

11. A device (1) for prostheses comprising:
- at least a bone integration portion (2) having an osseointegration surface (3) and a fixing surface (4), and adapted to be implanted in a skeletal structure;
- at least a contact portion (6) associated with said fixing surface (4) and having a contact surface (6a) adapted to engage with a joint element for the reconstruction of the joint of a patient;
**characterized by** the fact that said integration portion (2) comprises anchoring means (7) adapted to retain said contact portion (6) for their irreversible fixing.

12. The device (1) for prostheses according to claim 11, **characterized by** the fact that said anchoring means (7) comprise retaining undercuts (8) made on said fixing surface (4) adapted to retain said contact portion (6).

13. The device (1) for prostheses according to one or more of claims 11 and 12, **characterized by** the fact that said integration portion (2) comprises osseointegration means adapted to attract bone-growing cells.

14. The device (1) for prostheses according to one or more of claims 11 to 13, **characterized by** the fact that said osseointegration surface (3) comprises protrusions (5) of bone integration arranged at a predefined distance to one another.
